# EUROPEAN PATENT SPECIFICATION

(11) **EP 1 404 852 B1**
(45) Date of publication and mention of the grant of the patent: **21.12.2005**
(21) Application number: 03732369.8
(22) Date of filing: 14.05.2003
(51) Int. Cl.: C12N 15/86

(54) **EXPRESSION OF GENES IN MODIFIED VACCINIA VIRUS ANKARA BY USING THE COWPOX ATI PROMOTER**
EXPRESSION VON GENEN IM MODIFIZIERTEN VACCINIA VIRUS ANKARA DURCH VERWENDUNG EINES COWPOX ATI PROMOTER
EXPRESSION DES GENES DU VIRUS VACCINAL MODIFIE ANKARA PAR UTILISATION DU PROMOTEUR VACCINAL ATI

(30) Priority: 16.05.2002 DK 200200754; 25.11.2002 DK 200201813
(43) Date of publication of application: 07.04.2004
(73) Proprietor: Bavarian Nordic A/S, 3490 Kvistgaard (DK)
(72) Inventor: HOWLEY, Paul, Glen Waverly, VIC 3150 (AU); LEYRER, Sonja, 81377 München (DE)
(74) Representative: Alt, Michael
(86) International application number: PCT/EP2003/005046
(87) International publication number: WO 2003/097844

(56) References cited:
- EP-A- 0 330 781
- US-A- 5 225 336
- US-A- 5 426 051
- US-A- 5 453 364

## Description

The invention concerns recombinant Modified vaccinia virus Ankara comprising in the viral genome an expression cassette comprising the cowpox ATI promoter or a derivative thereof and a coding sequence, wherein the expression of the coding sequence is regulated by said promoter. The virus may be useful as a vaccine or as part of a pharmaceutical composition.

### Background of the invention

Recombinant poxviruses are widely used to express foreign antigens in infected cells. Moreover, recombinant poxviruses are currently tested as very promising vaccines to induce an immune response against the foreign antigen expressed from the poxvirus vector. Most popular are avipoxviruses on the one side and vaccinia viruses on the other side. US 5,736,368 and US 6,051,410 disclose recombinant vaccinia virus strain Wyeth that expresses HIV antigens and proteins. US 5,747,324 discloses a recombinant vaccinia virus strain NYCBH expressing lentivirus genes. EP 0 243 029 discloses a recombinant vaccinia virus strain Western Reserve expressing human retrovirus genes.

For the expression of heterologous genes in pox viruses several promoters are known to the person skilled in the art, such as the 30K and 40K promoters (see e.g. US 5,747,324), a strong synthetic early/late promoter (see e.g. Sutter et al., Vaccine (1994) 12, 1032.40), the P7.5 promoter (see e.g. Endo et al., J. Gen. Virol. (1991) 72, 699-703) and the promoter derived from the cowpox virus A-type inclusion (ATI) gene (Li et al., J. Gen. Virol. (1998) 79, 613). All of these promoters have been used in recombinant vaccinia viruses to express heterologous genes and were shown to express said genes very efficiently resulting in relatively high amounts of the protein encoded by the heterologous gene.

For many vaccination approaches it is highly desired that the antigen against which an immune response is to be induced is expressed in high amounts. However, this is not always the case. It has been described that different types of cytotoxic T-cells (CTL) are induced by the immune system depending on the concentrations of the antigen. Low avidity CTL's are induced by high concentrations of antigen, whereas high avidity CTL's are induced by low concentrations of antigen. It has been shown that high avidity CTL are much more effective at clearing the challenging virus in animal test systems than low avidity CTL. Moreover, it has been demonstrated that high concentrations of antigen might inhibit or even kill high avidity CTL. In summary, it has been shown that it is sometimes desirable to use rather low amounts of antigen to induce larger amounts of high avidity CTL and, thus, to induce an effective immune response (Berzofsky et al., Immunological Reviews (1999) 170, 151-172).

### Object of the invention

It was the object of the present invention to provide a vaccinia virus based system allowing the expression of a heterologous gene comprised in the vaccinia virus genome in relatively low amounts after administration to an animal, including a human being, which might be a prerequisite for the induction of relatively high amounts of high avidity CTL.

### Detailed description of the invention

This object has been solved by the provision of recombinant Modified vaccinia virus Ankara (MVA) comprising in the viral genome an expression cassette comprising the cowpox ATI promoter or a derivative thereof and a coding sequence, wherein the expression of the coding sequence is regulated by said promoter. It was unexpected that the ATI promoter has a relatively low activity in MVA since said promoter was known to be very active in other poxvirus systems (e.g. Li et al., J. Gen. Virol. (1998) 79, 613). In contrast, it is shown in the examples section of the present description that the ATI promoter is two to four times less active in MVA based systems than in systems based on other vaccinia virus strains such as Western Reserve, Elstree or Copenhagen. Thus, the ATI promoter in MVA is a good promoter for expressing genes encoding proteins against which high avidity CTL are to be induced.

Modified Vaccinia Ankara (MVA) virus is related to vaccinia virus, a member of the genera Orthopoxvirus in the family of Poxviridae. MVA has been generated by 516 serial passages on chicken embryo fibroblasts of the Ankara strain of vaccinia virus (CVA) (for review see Mayr, A., *et al.* Infection 3, 6-14 [1975]). As a consequence of these long-term passages the resulting MVA virus deleted about 31 kilobases of its genomic sequence and, therefore, was described as highly host cell restricted to avian cells (Meyer, H. *et al.*, J. Gen. Virol. 72, 1031-1038 [1991]). It was shown, in a variety of animal models that the resulting MVA was significantly avirulent (Mayr, A. & Danner, K. [1978] Dev. Biol. Stand. 41: 225-34). Additionally, this MVA strain has been tested in clinical trials as vaccine to immunize against the human smallpox disease (Mayr *et al.*, Zbl. Bakt. Hyg. I, Abt. Org. B 167, 375-390 [1987], Stickl *et al.*, Dtsch. med. Wschr. 99, 2386-2392 [1974]).

According to the present invention any MVA strain may be used. Examples for MVA virus strains used according to the present invention and deposited in compliance with the requirements of the Budapest Treaty are strains MVA 572 and 575 deposited at the European Collection of Animal Cell Cultures (ECACC), Salisbury (UK) with the deposition number ECACC V94012707 and ECACC V00120707, respectively and MVA-BN with the deposition number ECACC V00083008.

The most preferred MVA-strain is MVA-BN or a derivative thereof. The features of MVA-BN, the description of biological assays allowing to evaluate whether a MVA strain is MVA-BN or a derivative thereof and methods allowing to obtain MVA-BN or a derivative thereof are disclosed in WO 02/42480. The content of this application is included in the present application by reference.

In order to propagate MVA eukaryotic cells are infected with the virus. The eukaryotic cells are cells that are susceptible to infection with the respective poxvirus and allow replication and production of infectious virus. For MVA an example for this type of cells are chicken embryo fibroblasts (CEF) and BHK cells (Drexler I., Heller K., Wahren B., Erfle V. and Sutter G. "Highly attenuated modified vaccinia Ankara replicates in baby hamster kidney cells, a potential host for virus propagation, but not in various human transformed and primary cells" J. Gen. Virol. (1998), 79, 347.352). CEF cells can be cultivated under conditions known to the person skilled in the art. Preferably the CEF cells are cultivated in serum-free medium in stationary flasks or roller bottles. The incubation preferably takes place 48 to 96 hours at 37° C ± 2° C. For the infection MVA is preferably used at a multiplicity of infection (MOI) of 0,05 to 1 TCID₅₀ and the incubation preferably takes place 48 to 72 hours at 37 °C ± 2° C.

The sequence of the promoter of the cowpox virus A-type inclusion protein gene (ATI promoter) is known to the person skilled in the art. In this context reference is made to the Genebank entry accession number DO0319. A preferred ATI promoter sequence is shown as SEQ ID.: No. 1 and is as follows:

According to the present invention it is possible to use the ATI promoter as specified in SEQ. ID.:No. 1 or to use a derivative of the ATI promoter, which may be a subsequence of the sequence according to SEQ. ID.:No. 1. The term "subsequence of the sequence according to SEQ. ID.:No. 1" refers to shorter fragments of the sequence of SEQ. ID.:No. 1 that are still active as a promoter, in particular as vaccinia virus late promoter. A typical fragment of the sequence of SEQ. ID.:No. 1 has a length of at least 10 nucleotides, more preferably of at least 15 nucleotides, even more preferably of at least 20 nucleotides, most preferably of at least 25 nucleotides of the sequence of SEQ. ID.:No. 1. The subsequence preferably may comprise nucleotides 25 to 29 of SEQ. ID.:No. 1, i.e. the sequence 5'-TAAAT-3' located at the 3' end of SEQ. ID.:No. 1. The subsequence may also comprise nucleotides 22 to 29 of SEQ. ID.:No. 1, i.e. the sequence 5'-TAATAAAT-3' located at the 3' end of SEQ. ID.:No. 1.

The promoter may be inserted upstream of a coding sequence in such a way that nucleotides 28 to 29 of SEQ. ID: 1 (underlined in the sequence above) are part of the 5 'ATG 3' start codon of translation. Alternatively, the promoter may be separated by several nucleotides from the start codon of translation.

The spacer between the 3' end of the promoter according to SEQ ID.: No 1 and the A in the 5' ATG 3' start codon is preferably less than 100 nucleotides, more preferably less than 50 nucleotides and even more preferably less than 25 nucleotides. However, the spacer might even be longer as long as the promoter is still capable of directing the expression of the coding sequence located downstream of the promoter.

The derivative of the ATI promoter can also be a sequence that has one or more nucleotide substitutions, deletions and/or insertions with respect to the sequence of SEQ ID.: No. 1, wherein said derivatives are still active as a promoter, in particular as vaccinia virus late promoter. A sequence having one or more nucleotide substitutions is a sequence in which one or more nucleotides of the sequence according to SEQ ID.: No. 1 are substituted by different nucleotides. A sequence having one or more nucleotide insertions is a sequence in which one or more nucleotides are inserted at one or more locations of the sequence according to SEQ ID.: No. 1. A sequence having one or more nucleotide deletions is a sequence in which one or more nucleotides of the sequence according to SEQ ID.: No. 1 are deleted at one or more locations. In the derivatives of SEQ ID.: No. 1 deletions, substitutions and insertions may be combined in one sequence.
Preferably the derivative has a homology of at least 40%, more preferably of at least 60%, even more preferably of at least 80%, most preferably of at least 90% when compared to the sequence of SEQ ID.: No.1. According to the most preferred embodiment not more than 6 nucleotides, even more preferably not more than 3 nucleotides are substituted, deleted and/or inserted in the sequence of SEQ ID: No. 1.
In particular, it might be preferable to keep nucleotides 25 to 29 of SEQ. ID.:No. 1, i.e. the sequence 5'-TAAAT-3' in the promoter to attain maximal promoter activity. It might also be preferable to keep nucleotides 22 to 29 of SEQ. ID.:No. 1, i.e. the sequence 5'-TAATAAAT-3 in the promoter.

A bundle of prior art documents allows the person skilled in the art to predict which derivatives of SEQ ID.: No. 1 still have the biological activity of being active as a vaccinia virus promoter, in particular as a vaccinia virus late promoter. In this context reference is made to Chakrarbarti et al., Biotechniques (1997) 23, 1094-1097 and Davison and Moss, J. Mol. Biol. (1989) 230, 771-784. Moreover, whether a fragment is still active as a vaccinia virus promoter, in particular a late promoter can easily be checked by a person skilled in the art. In particular the sequence derivative can be cloned upstream of a reporter gene in a plasmid construct. Said construct may be transfected into a eukaryotic cell or cell line, such as CEF or BHK cells that has been infected with MVA. The expression of the reporter gene is then determined and compared to the expression of the reporter gene controlled by the promoter according to SEQ ID.: No. 1. The experimental setting corresponds to the example shown in the present specification. A derivative according to the present invention is a derivative having a promoter activity in said test system of at least 10%, preferably at least 30%, more preferably at least 50%, even more preferably at least 70%, most preferably at 90% compared to the activity of the promoter sequence of SEQ ID.: No.1. Also those derivatives of SEQ ID.: No.1 are within the scope of the present invention that have a higher promoter activity than SEQ ID.: No. 1.

In more general terms the present invention refers to the use of the cowpox ATI promoter or a derivative thereof as defined above for the expression of coding sequences in MVA.

The ATI promoter may be used to express a gene that is already part of the MVA genome. Such a gene may be a gene that is naturally part of the viral genome or a foreign gene that has already been inserted into the MVA genome. In these cases the ATI promoter is inserted upstream of the gene in the MVA genome, the expression of which is to be controlled by the ATI promoter.

The ATI promoter may also be used to regulate the expression of a gene that is not yet part of the MVA genome. In this case it is preferred to construct an expression cassette comprising the ATI promoter and a coding sequence, the expression of which is to be regulated by the ATI promoter and to insert said expression cassette into the MVA genome. Preferred insertion sites are selected from (i) naturally occurring deletion sites of the MVA genome with respect to the genome of the vaccinia virus strain Copenhagen or (ii) intergenic regions of the MVA genome. The term "intergenic region" refers preferably to those parts of the viral genome located between two adjacent genes that comprise neither coding nor regulatory sequences. However, the insertion sites are not restricted to these preferred insertion sites since it is within the scope of the present invention that the expression cassette may be inserted anywhere in the viral genome as long as it is possible to obtain recombinants that can be amplified and propagated in at least one cell culture system, such as Chicken Embryo Fibroblasts (CEF cells). Thus, the insertion cassette may also be inserted e.g. into non-essential genes or genes the function of which may be supplemented by the cell system used for propagation of MVA.

The methods necessary to construct recombinant MVA are known to the person skilled in the art. By way of example, the expression cassette and/or the ATI promoter or derivative thereof may be inserted into the genome of MVA by homologous recombination. To this end a nucleic acid is transfected into a permissive cell line such as CEF or BHK cells, wherein the nucleic acid comprises the expression cassette and/or the ATI promoter or derivative thereof flanked by nucleotide stretches that are homologous to the region of the MVA genome in which the expression cassette and/or the ATI promoter or derivative thereof is to be inserted. The cells are infected by MVA and in the infected cells homologous recombination occurs between the nucleic acid and the viral genome. Alternatively it is also possible to first infect the cells with MVA and then to transfect the nucleic acid into the infected cells. Again recombination occurs in the cells. The recombinant MVA is then selected by methods known in the prior art. The construction of recombinant MVA is not restricted to this particular method. Instead, any suitable method known to the person skilled in the art may be used to this end.

The ATI promoter in MVA may be used to control the expression of any coding sequence. The coding sequence may preferably code for at least one antigenic epitope or antigen. In this case the recombinant MVA may be used to express said antigen after infection of cells in an organism, e.g. a mammalian animal including a human. The presentation of said antigen/epitope may elicit an immune response in the organism that may lead to a vaccination of the organism against the agent from which the antigen/epitope is derived. More specifically the epitope/antigen may be part of a larger amino acid sequence such as a polyepitope, peptide or protein. Examples for such polyepitopes, peptides or proteins may be polyepitopes, peptides or proteins derived from (i) viruses, such as HIV, HTLV, Herpesvirus, Denguevirus, Poliovirus, measles virus, mumps virus, rubella virus, Hepatitis viruses and so on, (ii) bacteria, (iii) fungi.

Alternatively the coding sequence may encode a therapeutic compound such as interleukins, interferons, ribozymes or enzymes.

The recombinant MVA may be administered to the animal or human body according to the knowledge of the person skilled in the art. Thus, the recombinant MVA according to the present invention may be useful as a medicament (i.e. pharmaceutical composition) or vaccine.

The pharmaceutical composition or the vaccine may generally include one or more pharmaceutical acceptable and/or approved carriers, additives, antibiotics, preservatives, adjuvants, diluents and/or stabilizers in addition to the recombinant MVA. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycollic acids, polymeric amino acids, amino acid copolymers, lipid aggregates, or the like.

For the preparation of pharmaceutical compositions or vaccines, the recombinant MVA is converted into a physiologically acceptable form. This can be done based on the experience in the preparation of poxvirus vaccines used for vaccination against smallpox (as described by Stickl, H. *et al.* [1974] Dtsch. med. Wschr. 99, 2386-2392). For example, the purified virus is stored at -80°C with a titre of 5x10⁸ TCID₅₀/ml formulated in about 10mM Tris, 140 mM NaCl pH 7.4. For the preparation of vaccine shots, e.g., 10¹-10⁹ particles of the recombinant virus according to the present invention are lyophilized in phosphate-buffered saline (PBS) in the presence of 2% peptone and 1% human albumin in an ampoule, preferably a glass ampoule. Alternatively, the vaccine shots can be produced by stepwise freeze-drying of the virus in a formulation. This formulation can contain additional additives such as mannitol, dextran, sugar, glycine, lactose or polyvinylpyrrolidone or other additives such as antioxidants or inert gas, stabilizers or recombinant proteins (e.g. human serum albumin) suitable for *in vivo* administration. A typical virus containing formulation suitable for freeze-drying comprises 10 mM Tris-buffer, 140 mM NaCl, 18.9 g/I Dextran (MW 36000 - 40000), 45 g/I Sucrose, 0.108 g/I L-glutamic acid mono potassium salt monohydrate pH 7.4. The glass ampoule is then sealed and can be stored between 4°C and room temperature for several months. However, as long as no need exists the ampoule is stored preferably at temperatures below 20°C.

For vaccination or therapy the lyophilisate or the freeze-dried product can be dissolved in 0.1 to 0.5 ml of an aqueous solution, preferably water, physiological saline or Tris buffer, and administered either systemically or locally, i.e. by parenteral, intramuscular or any other path of administration know to the skilled practitioner. The mode of administration, the dose and the number of administrations can be optimized by those skilled in the art in a known manner.

Thus, according to a related embodiment the invention relates to a method for affecting, preferably inducing an immunological response in a living animal body including a human comprising administering the virus, the composition or the vaccine according to the present invention to the animal or human to be treated. Typically, a vaccine shot comprises at least 10², preferably at least 10⁴, more preferably at least 10⁶, even more preferably 10⁸ TCID₅₀ (tissue culture infectious dose) of the virus.

It is a particular advantage of the recombinant MVA according to the present invention, in particular of recombinant MVA-BN and its derivatives that the virus may be used for prime-boost administration. Thus, the invention further relates to a method, wherein the virus, the composition or the vaccine is administered to an animal, including a human in need thereof, in therapeutically effective amounts in a first inoculation ("priming inoculation") and in a second inoculation ("boosting inoculation").

The invention further concerns a method for introducing a coding sequence into target cells comprising the infection of the target cells with the virus according to the present invention. The target cell may be a cell in which the virus is able to replicate such as CEF or BHK cells or a cell that can be infected by MVA, in which the virus, however, does not replicate, such as all types of human cells.

The invention further relates to a method for producing a peptide, protein and/or virus comprising the infection of a host cell with a recombinant virus according to the present invention, followed by the cultivation of the infected host cell under suitable conditions, and further followed by the isolation and/or enrichment of the peptide and/or protein and/or viruses produced by said host cell. If it is intended to produce, i.e. amplify the virus according to the present invention the cell has to be a cell in which the virus is able to replicate such as CEF or BHK cells. If it is intended to produce a peptide/protein encoded by the virus, preferably a protein/peptide encoded by a coding sequence, the expression of which is controlled by the ATI promoter or a derivative thereof, the cell may be any cell that can be infected by the recombinant virus and that allows the expression of MVA encoded proteins/peptides.

The invention further relates to cells infected with the virus according to the present invention.

### Short Description of the Figures

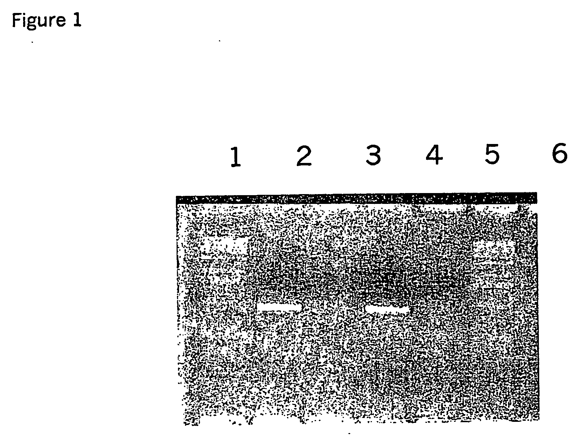

**Figure 1:** Results of a PCR reaction to detect the presence of RNA expressed from the ATI promoter in a recombinant MVA after infection of CEF cells (see Example 2)

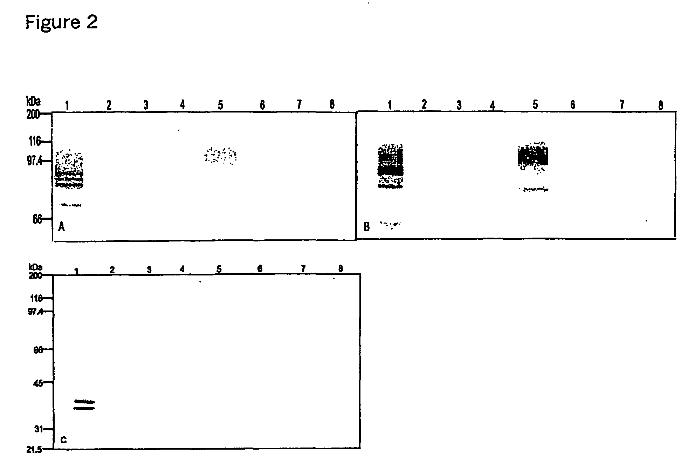

**Figure 2:** Results of a Western blot with proteins isolated from cells infected with various recombinant MVAs. Figure 2A: non-reducing conditions, non-heated proteins; Figure 2 B: reducing conditions, non-heated proteins; C-non-reducing conditions, heated proteins. Lanes 1, 3, 4 are the cell lysates of cells infected with MVA-ATI-NS1, MVA-GFP as well as uninfected cells, respectively. Lanes 5, 7, 8 are the supernatants of cells infected with MVA-ATI-NS1, MVA-GFP and the supernatants of cell controls, respectively. Lanes 2 and 6 have been left empty.

### Examples

The following example(s) will further illustrate the present invention. It will be well understood by a person skilled in the art that the provided example(s) in no way may be interpreted in a way that limits the applicability of the technology provided by the present invention to this example(s).

### Example 1: Activity of the cowpox ATI promoter in different vaccinia virus strains

The aim of this example was to analyze the strength of the cowpox ATI promoter in different vaccinia virus strains.

### Introduction:

The cowpox ATI promoter was fused to the GUS (E. coli β-Glucuronidase) reporter gene for expression analysis. BHK (baby hamster kidney) cells were infected with different vaccinia virus strains and transfected with a plasmid containing the ATI promoter fused to the GUS gene. The analyzed vaccinia virus strains comprised CVA, Copenhagen, Elstree, IHD, Western reserve and MVA-BN. If the promoter was functional, GUS would be expressed and could be quantified by an enzymatic reaction.

### Materials and Equipment:

- BHK cells (ECACC No. 84100501)
- All vaccinia viruses were used with a titer of 7.5 x 10⁷ TCID₅₀ per ml
- Plasmid pBNX73 (pBluescript + ATI promoter + GUS)
- Effectene transfection kit (Qiagen)
- DMEM cell culture media (Gibco BRL)
- FCS (Gibco BRL)
- Lysisbuffer (PBS + 0.1 % Triton + 1 mM protease inhibitor)
- GUS substrate 1mM (p-Nitrophenyl-beta-(D)-glucuronide; Sigma, Cat. No. N1627)
- Stop solution 2.5 M (2-amino-2-methyl-1,3-propandiol; Sigma, Cat. No. A 9754)

### Method:

### Seeding of cells

5 x 10⁵ BHK cells were seeded per transfection reaction in a well of a 6-well-plate and maintained in DMEM/10%FCS over night at 37°C and 5% CO₂.

### Infection/transfection

Cells were infected with the different vaccinia virus strains (MOI 0.1) in 0.5 ml DMEM/10% FCS per well and incubated for 1 h at room temperature on a shaker. Transfection was performed as described in the manufacturers protocol. 2 µg plasmid were diluted in buffer EB (100 µl total volume). After addition of 3.2 µl enhancer solution the solution was mixed and incubated for 5 min. at room temperature. Then 10 µl Effectene reagen was added, suspension was mixed and incubated for 10 min. at room temperature. The virus-suspension was removed from the cells and 1.6 ml DMEM/10%FCS were added. 0.6 ml DMEM/10%FCS were added to the DNA Effectene mixture and dropped on the cells while rotating the culture plate. Cells were then incubated for 48 hours.

### Harvesting of the cells

Medium was removed from cells and 0.5 ml of Lysis buffer was added. After shaking 15 min. at RT, cells were scraped in the Lysis buffer, transferred to a 1.5 ml reaction tube and vortexed vigorously. Lysed cells were centrifuged for 1 min. at 500 rcf and 4°C, the clear supernatant was transferred to a fresh vial and stored at -20°C until use.

### Determination of GUS activity

10 µl of cell extract (= protein out of 2 x 10⁴ cells) was added to 1 ml pre-warmed substrate solution (37°C) and incubated at 37°C until a yellow colour was developed. Samples were then placed on ice immediately and 0.4 ml stop solution was added. The extinction at 415 nm was determined and equated with the GUS activity as extinction values between 0.05 and 2.0 are in a linear range. The substrate solution was used as reference and a cell extract of uninfected cells was used as negative control.

### Results:

The quantification of the GUS activity expressed from the ATI promoter- GUS gene construct in BHK cells infected with the different vaccinia virus strains gave the following results:

| Vaccinia virus strain used for infection of BHK cells transfected with pBNX73 | GUS activity |
|---|---|
| (uninfected cells) | 0 |
| CVA | 1.30 |
| Copenhagen | 1.86 |
| Elstree | 2.07 |
| IHD | 1.30 |
| Western Reserve | 0.96 |
| MVA-BN | 0.48 |

### Example 2: Expression of foreign genes inserted in the MVA genome and regulated by the Cowpox ATI promoter

The aim of this example was to demonstrate that the ATI promoter is capable to regulate and to express genes when inserted in the genome of MVA.

### Introduction:

The cowpox ATI promoter was fused to the non-structural (NS) 1 gene of Dengue virus. This expression cassette was inserted into a recombination vector comprising sequences homologous to the MVA genome. In the resulting recombination plasmid the expression cassette was flanked by sequences homologous to the sequences in the MVA genome in which the expression cassette was to be inserted. CEF cells were infected with MVA-BN and transfected with the recombination vector comprising the ATI-promoter NS1 expression cassette. In the cells homologous recombination occurred between the MVA genome and the recombination plasmid resulting in a recombinant MVA genome. After several rounds of purification it was analyzed whether the NS1 protein in the recombinant MVA was expressed from the ATI promoter. In parallel experiments an expression cassette comprising a sequence encoding a HIV polytope under the control of the ATI promoter was inserted into the MVA genome and it was again tested whether the ATI promoter is active in MVA and expresses the HIV polytope.

### Materials and Equipment:

- primary CEF cells
- Baby hamster kidney cells (BHK; deposit 85011433 at the European Collection of Animal Cell Cultures)
- MVA-BN with a titre of 10⁸ TCID₅₀/ml.
- plasmid pBN74 and pBN84: pBN74 comprises a sequence encoding an HIV-polytope under the control of an ATI promoter and pBN84 comprises the coding sequence for the Denguevirus NS1 protein under the control of the ATI promoter. In addition to the expression cassettes comprising the Denguevirus coding sequence and HIV polytope coding sequence, respectively, both plasmids comprise a G418 resistance gene and a gene coding for the green fluorescent protein as marker genes. The marker genes as well as the expression cassettes for Denguevirus NS1 and the HIV polytope, respectively, are flanked by MVA sequences that are homologous to the region of the MVA genome in which the heterologous genes are to be inserted.
- Effectene transfection kit (Qiagen)
- VP-SFM cell culture media (Gibco BRL)
- RNeasy RNA isolation kit (Qiagen)
- DNAse, RNAse free (Roche)
- MMLV Reverse transcriptase (Promega)
- Taq DNA polymerase (Roche)
- RNAse inhibitor RNAsin (Promega)
- The following oligosnucleotides were purchased from MWG, Germany: primer oBN465 ggtctgatttccatcccgtac (21 nucleotides), used for the reverse transcriptase reaction; primer oBN463 gaactgaagtgtggcagt (18 nucleotides), used for PCR; primer oBN464 cggtggtaatgtgcaagatc (20 nucleotides), used for PCR

### Methods

### Integration into MVA Genome by Homologous Recombination

The above described plasmids pBN74 or pBN84 were used to integrate a HIV polytope coding sequence and the dengue NS1 expression cassette, respectively, into the MVA genome by homologous recombination between MVA sequences flanking the expression cassettes in pBN74 or pBN84 on the one side and the homologous target sequences within the MVA genome on the other side. This is achieved by transfecting the linearized plasmid pBN74 or pBN84 into chicken embryo fibroblast (CEF) cells previously infected with MVA at low multiplicity of infection. More specifically, CEF cells were seeded in 6-well-plates and maintained in VP-SFM over night at 37°C and 5% CO₂. The cells were infected with MVA-BN (MOI 1.0) in 0.5 ml VP-SFM per well and incubated for 1 h at room temperature on a shaker. Transfection of the cells either with pBN74 or pBN84 was performed as described in the manufacturer protocol. At 48 hours post infection or when the infection had reached confluency a viral extract is prepared and stored at -20°C ready for selection and clone purification of desired recombinant MVA (rMVA).

### Selection of recombinant MVA (rMVA) and Clone Purification

The elimination of non-recombinant MVA (empty vector virus) and the amplification of rMVA is achieved by infection of confluent chicken embryo fibroblast (CEF) cells at a low MOI in the presence of G418 (amount of G418 has to be optimized to determine the highest dose that dose not kill the CEF cells). Any virus that does not contain an integrated NPT II gene will not replicate in the presence of G418 added to the cell maintenance medium. G418 inhibits DNA replication but since the CEF cells will be in the stationary non-replicating state they will not be affected by the action of G418. CEF cells infected with rMVAs can be visualized under a fluorescence microscope due to the expression of the enhanced fluorescent green protein.

Viral extracts from the homologous recombination step are serially diluted and are used to infect fresh CEF cells in the presence of G418. The infected cells are overlaid with low-melting point agarose. After 2 days of infection, the plates are observed under a fluorescent microscope for single foci of green infected cells. These cells are marked and agarose plugs containing the infected foci of cells are taken and placed into 1.5 ml microcentrifugation tubes containing sterile cell maintenance medium. Virus is released from the agarose plug by freeze-thawing the tube three times at - 20°C.

The recombinant MVA with the inserted dengue NS1 expression cassette described in this invention was termed MVA-ATI-NS1.

### Detection of RNA expressed from the ATI-promoter in recombinant MVA

For the detection of RNA expressed from the ATI-promoter in cells infected with the recombinant MVA, RNA extraction was performed as described in the manufacturers protocol (Rneasy Mini Protocol for the Isolation of Total RNA from Animal Cells). A DNAse digestion reaction was performed by adding 3 µl DNAse RNase free (= 30U; Roche), 3 µl 10 x buffer A (usually used for restrictions, Roche) to 5 µg RNA in a volume of 30 µl adjusted with water. The mixture was incubated for 90' at 37°C. The RNA was purified by using Rneasy columns according to the manufacturer's protocol. For reverse transcription 2 µg RNA were mixed with 1 µg primer oBN465 for reverse transcription and the total volume was adjusted to 10 µl by adding water. It was incubated for 5' at 70°C and the tube was transferred to ice. Then 5 µl 5 x buffer, 5 µl dNTP Mix (10 µM), 0.5 µl Rnasin, 2 µl M-MLV RT (200U) and 2.5 H₂O were added and the mixture was incubated for 60'at 42°C

For the PCR amplification 5 µl of the RT reaction was mixed with 36 µl H₂O, 5 µl 10 x buffer, 1 µl dNTPs, 2 µl of each primer oBN463 and oBN464 (10 µM) and 1 µl Taq-polymerase. The DNA was amplified in 25 cycles (1 min extension, annealing temperature 55 °C) and analyzed by gel electrophoresis.

### Detection of Denguevirus NS1 protein expressed in cells infected with MVA-ATI-NS1

A 25 cm² flask with about 80% confluent monolayers of BHK cells was inoculated with 100µl of MVA-ATI-NS1 virus stock diluted to 1x10⁷ in MEMα with 1%FCS and rocked at room temperature for 30 minutes. 5 ml of MEMα with 3%FCS were added to each flask and incubated at 30°C in a CO₂ incubator. The flask was harvested after 48 hours. The supernatant was removed from the flask and spun at 260g for 10 minutes at 4°C. The supernatants were stored in aliquots at -80°C. The pellet was washed two times with 5 ml of 1x PBS and then resuspended in 1ml of hypotonic douncing buffer with 1%TX100. The cell lysates were harvested and spun for 5 minutes at 16,000g and the supernatants were stored in a microcentrifuges tube at -80°C.

Flasks inoculated with control viruses and uninfected flasks were also treated the same way as described above.

The cell/viral lysate and the supernatant were treated in either a non-reducing or reducing sample buffer either under non-heated or heated conditions. The proteins were separated on 10% SDS PAGE and transferred to nitrocellulose membranes. The blots were probed overnight with pooled sera from convalescent patient (PPCS), i.e. sera from patients that suffered from a Denguevirus infection, at 1:500 dilution. After washing 3 times with 1X PBS the blots were incubated with anti-human IgG-horse radish peroxidase (HRP) (DAKO) for 2 hours at room temperature. After the blots were washed as described before the colour was developed using 4 chloro-1-napthol. The results are shown in figure 2.

### Results

The NS1 gene as well as the HIV polytope were shown to be expressed, when regulated by the Cowpox ATI promoter (Figure 1). The corresponding mRNA was clearly detectable. More particularly, the expected signal of 926 bp was clearly detectable after RT-PCR of the RNA sample (Figure 1, lane 2). Using the sample for PCR only, no signal was detectable (Figure 1, lane 3). Therefore a false positive signal caused by DNA contamination can be excluded. Figure 1, lane 4 shows the result of the PCR with the plasmid positive control. As expected the size of the PCR product was identical to the size of the PCR product after RT-PCR of the RNA sample. Figure 1, lane 5 shows the result of an RT-PCT reaction with a negative control (water). Figure 1, lanes 1 and 6 are a molecular weight marker (100bp ladder).

The western blots results showed that NS1 is expressed in cells infected with MVA-ATI-NS1. NS1 was expressed in the right conformation and as a dimer under non-heated conditions as shown in lane 1 of figure 2 A and 2 B. When the sample was heated the NS1 monomer can be seen as shown in Figure 2 C.

The results also showed that NS1 expressed in cells infected with MVA-ATI-NS1 is antigenic and is recognized by the pooled convalescent patients' sera.

In conclusion, the experiments have shown that NS1 is expressed in the right conformation in the BHK cells infected with MVA-ATI-NS1. Both the dimer and monomer are antigenic and is recognized by the pooled convalescent patients' sera.

## Claims

1. Recombinant Modified vaccinia virus Ankara (MVA) comprising in the viral genome an expression cassette comprising the cowpox ATI promoter or a derivative thereof and a coding sequence, wherein the expression of the coding sequence is regulated by said promoter or derivative thereof and wherein the derivative of the ATI promoter is
(i) a sequence having a homology of at least 60% when compared to the sequence of SEQ ID: No. 1,
(ii) a sequence in which not more than 6 nucleotides are substituted, deleted and/or inserted in the sequence of SEQ ID: No.1 and/or
(iii) a subsequence of the sequence according to SEQ ID: No. 1, wherein said subsequence has a length of at least 10 nucleotides of the sequence of SEQ ID: No. 1
wherein said sequence and subsequence of (i), (ii) and/or (iii) are still active as a promoter.

2. Recombinant MVA according to claim 1, wherein the sequence and subsequence according to (i), (ii) and/or (iii) are still active as a Vaccinia virus late promoter.

3. Recombinant MVA according to anyone of claims 1 to 2, wherein the subsequence of (iii) has a length of at least 20 nucleotides of the sequence of SEQ ID: No. 1.

4. Recombinant MVA according to anyone of claims 1 to 3, wherein the subsequence of (iii) has a length of at least 25 nucleotides of the sequence of SEQ ID: No. 1.

5. Recombinant MVA according to claim 1, wherein the sequence of (i) have a homology of at least 80% when compared to the sequence of SEQ ID: No. 1.

6. Recombinant MVA according to anyone of claims 1 to 5, wherein the sequence of (i), (ii) and/or the subsequence of (iii) comprise nucleotides 25 to 29 and/or 22 to 29 of SEQ I D: No. 1

7. Recombinant MVA according to claim 1, wherein the ATI promoter has the sequence of SEQ I D: No. 1

8. Recombinant MVA according to anyone of claims 1 to 7, wherein MVA is selected from strain MVA-BN deposited at the European Collection of Cell Cultures (ECACC) under number V00083008 or a derivative thereof and strain MVA 575 deposited under number V00120707 at ECACC.

9. Recombinant MVA according to anyone of claims 1 to 8, wherein the expression cassette is inserted in a naturally occurring deletion site of the MVA genome with respect to the genome of the vaccinia virus strain Copenhagen or in an intergenic region of the MVA genome.

10. Recombinant MVA according to anyone of claims 1 to 9, wherein the coding sequence codes for least one antigen, antigenic epitope, and/or a therapeutic compound.

11. Recombinant MVA according to anyone of claims 1 to 10 as vaccine or medicament.

12. Vaccine or pharmaceutical composition comprising a recombinant MVA according to anyone of claims 1 to 10.

13. Use of the recombinant MVA according to anyone of claims 1 to 10 for the preparation of a vaccine or medicament to elicit an immune response against the agent from which the antigen/epitope encoded by the coding sequence is derived.

14. Use according to claim 13, wherein the antigen/epitope is part of a polypeptide, peptide or protein derived from a virus, bacterium or fungus.

15. Use according to claim 14, wherein the virus is selected from HIV, HTLV, Herpesvirus, Denguevirus, Poliovirus, measles virus, mumps virus, rubella virus and Hepatitis viruses.

16. Vaccine or pharmaceutical composition according to claim 12 or use according to anyone of claims 13 to 15, wherein the vaccine, the pharmaceutical composition or the medicament comprises at least 10² TCID₅₀ (tissue culture infectious dose) of the virus.

17. Vaccine or pharmaceutical composition according to anyone of claims 12 or 16 or use according to anyone of claims 13 to 15, wherein the vaccine, the pharmaceutical composition or the medicament is to be administered in therapeutically effective amounts in a first inoculation ("priming inoculation") and in a second inoculation ("boosting inoculation").

18. Method for introducing a coding sequence into target cells comprising the infection of the target cells with the virus according to anyone of claims 1 to 10, wherein the target cell is not a cell in the human and/or animal body.

19. Method for producing a peptide, protein and/or virus comprising
a) infection of a host cell with the virus according to anyone of claims 1 to 10,
b) cultivation of the infected host cell under suitable conditions, and
c) isolation and/or enrichment of the peptide and/or protein and/or viruses produced by said host cell.

20. A cell containing the virus according to any of claims 1 to 10.

21. Use of the cowpox ATI promoter or a derivative thereof as defined in anyone of claims 1 to 7 for the expression of coding sequences in MVA.

22. Method for the production of a recombinant MVA according to anyone of claims 1 to 10 comprising the step of inserting an expression cassette into the genome of MVA.

## Patentansprüche

1. Rekombinantes modifiziertes Vacciniavirus Ankara (MVA), dessen Virusgenom eine den Cowpox-ATI-Promotor oder ein Derivat davon umfassende Expressionskassette umfaßt, wobei die Expression der codierenden Sequenz durch den Promotor oder dessen Derivat reguliert wird und wobei es sich bei dem Derivat des ATI-Promotors um
(i) eine Sequenz mit einer Homologie von mindestens 60% im Vergleich mit der Sequenz von SEQ ID: Nr. 1,
(ii) eine Sequenz, in der höchstens 6 Nukleotide in der Sequenz von SEQ ID: Nr. 1 substituiert, deletiert und/oder inseriert sind, und/oder
(iii) eine Teilsequenz der Sequenz gemäß SEQ ID: Nr. 1, wobei die Teilsequenz eine Länge von mindestens 10 Nukleotiden der Sequenz von SEQ ID: Nr. 1 aufweist,
handelt, wobei die Sequenz und Teilsequenz aus (i), (ii) und/oder (iii) noch als Promotor aktiv sind.

2. Rekombinantes MVA nach Anspruch 1, wobei die Sequenz und Teilsequenz gemäß (i), (ii) und/oder (iii) noch als später Vacciniavirus-Promotor aktiv sind.

3. Rekombinantes MVA nach einem der Ansprüche 1 bis 2, wobei die Teilsequenz aus (iii) eine Länge von mindestens 20 Nukleotiden der Sequenz von SEQ ID: Nr. 1 aufweist.

4. Rekombinantes MVA nach einem der Ansprüche 1 bis 3, wobei die Teilsequenz aus (iii) eine Länge von mindestens 25 Nukleotiden der Sequenz von SEQ ID: Nr. 1 aufweist.

5. Rekombinantes MVA nach Anspruch 1, wobei die Sequenz aus (i) eine Homologie von mindestens 80% im Vergleich mit der Sequenz von SEQ ID: Nr. 1 aufweist.

6. Rekombinantes MVA nach einem der Ansprüche 1 bis 5, wobei die Sequenz aus (i), (ii) und/oder die Teilsequenz aus (iii) die Nukleotide 25 bis 29 und/oder 22 bis 29 von SEQ ID: Nr. 1 umfaßt.

7. Rekombinantes MVA nach Anspruch 1, wobei der ATI-Promotor die Sequenz von SEQ ID: Nr. 1 aufweist.

8. Rekombinantes MVA nach einem der Ansprüche 1 bis 7, wobei das MVA ausgewählt ist aus dem unter der Nummer V00083008 bei der European Collection of Cell Cultures (ECACC) hinterlegten Stamm MVA-BN oder einem Derivat davon sowie dem unter der Nummer V00120707 bei der ECACC hinterlegten Stamm MVA 575.

9. Rekombinantes MVA nach einem der Ansprüche 1 bis 8, wobei die Expressionskassette an einer natürlich vorkommenden Deletionsstelle des MVA-Genoms, bezogen auf das Genom des Vacciniavirusstamms Copenhagen, oder in einem Zwischengenbereich des MVA-Genoms inseriert ist.

10. Rekombinantes MVA nach einem der Ansprüche 1 bis 9, wobei die codierende Sequenz für mindestens ein Antigen, antigenes Epitop und/oder eine therapeutische Verbindung codiert.

11. Rekombinantes MVA nach einem der Ansprüche 1 bis 10 als Impfstoff oder Arzneimittel.

12. Impfstoff oder pharmazeutische Zusammensetzung, umfassend ein rekombinantes MVA nach einem der Ansprüche 1 bis 10.

13. Verwendung des rekombinanten MVA nach einem der Ansprüche 1 bis 10 zur Herstellung eines Impfstoffs oder Arzneimittels zum Hervorrufen einer Immunantwort gegen das Agens, aus dem das von der codierenden Sequenz codierte Antigen/Epitop stammt.

14. Verwendung nach Anspruch 13, wobei das Antigen/Epitop Teil eines aus einem Virus, Bakterium oder Pilz stammenden Polypeptids, Peptids oder Proteins ist.

15. Verwendung nach Anspruch 14, wobei das Virus ausgewählt ist aus HIV, HTLV, Herpesvirus, Denguevirus, Poliovirus, Masernvirus, Mumpsvirus, Rubellavirus und Hepatitisviren.

16. Impfstoff oder pharmazeutische Zusammensetzung nach Anspruch 12 oder Verwendung nach einem der Ansprüche 13 bis 15, wobei der Impfstoff, die pharmazeutische Zusammensetzung oder das Arzneimittel mindestens 10² TCID₅₀ (infektiöse Gewebekulturdosis) des Virus umfaßt.

17. Impfstoff oder pharmazeutische Zusammensetzung nach einem der Ansprüche 12 oder 16 oder Verwendung nach einem der Ansprüche 13 bis 15, wobei der Impfstoff, die pharmazeutische Zusammensetzung oder das Arzneimittel in therapeutisch wirksamen Mengen in einer ersten Inokulation ("Primer-Inokulation") und in einer zweiten Inokulation ("Booster-Inokulation") zu verabreichen ist.

18. Verfahren zur Einführung einer codierenden Sequenz in Zielzellen, bei dem man die Zielzellen mit dem Virus nach einem der Ansprüche 1 bis 10 infiziert, wobei es sich bei der Zielzelle nicht um eine Zelle im menschlichen und/oder tierischen Körper handelt.

19. Verfahren zur Herstellung eines Peptids, Proteins und/oder Virus, wobei man in dem Verfahren
a) eine Wirtszelle mit dem Virus nach einem der Ansprüche 1 bis 10 infiziert,
b) die infizierte Wirtszelle unter geeigneten Bedingungen kultiviert und
c) das von der Wirtzelle produzierte Peptid und/oder Protein und/oder die von der Wirtzelle produzierten Viren isoliert und/oder anreichert.

20. Zelle, enthaltend das Virus nach einem der Ansprüche 1 bis 10.

21. Verwendung des Cowpox-ATI-Promotors oder eines Derivats davon mit der in einem der Ansprüche 1 bis 7 angegebenen Bedeutung zur Expression von codierenden Sequenzen in MVA.

22. Verfahren zur Herstellung eines rekombinanten MVA nach einem der Ansprüche 1 bis 10, wobei man in einem Verfahrensschritt eine Expressionskassette in das MVA-Genom inseriert.

## Revendications

1. Virus de la vaccine Ankara modifié (MVA) recombinant comprenant, dans le génome viral, une cassette d'expression qui comprend le promoteur ATI de la vaccine ou un dérivé de celui-ci et une séquence codante, dans lequel l'expression de la séquence codante est régulée par ledit promoteur ou un dérivé de celui-ci et dans lequel le dérivé du promoteur ATI est :
(i) une séquence ayant une homologie de 60% au moins, quand elle est comparée à la séquence de la SEQ ID n° : 1,
(ii) une séquence dans laquelle au maximum 6 nucléotides sont substitués, ont subi une délétion et/ou sont insérés dans la séquence de la SEQ ID n° : 1 et/ou
(iii)une sous-séquence de la séquence selon la SEQ ID n° : 1, dans laquelle ladite sous-séquence a une longueur de 10 nucléotides au moins de la séquence de la SEQ ID n° : 1
dans lequel lesdites séquence et sous-séquence des paragraphes (i), (ii) et/ou (iii) sont encore actives en tant que promoteur.

2. MVA recombinant selon la revendication 1, dans lequel la séquence et la sous-séquence selon les paragraphes (i), (ii) et/ou (iii) sont encore actives en tant que promoteur tardif du virus de la vaccine.

3. MVA recombinant selon l'une quelconque des revendications 1 à 2, dans lequel la sous-séquence du paragraphe (iii) a une longueur de 20 nucléotides au moins de la séquence de la SEQ ID n° : 1.

4. MVA recombinant selon l'une quelconque des revendications 1 à 3, dans lequel la sous-séquence du paragraphe (iii) a une longueur de 25 nucléotides au moins de la séquence de la SEQ ID n° : 1.

5. MVA recombinant selon la revendication 1, dans lequel la séquence du paragraphe (i) a une homologie de 80% au moins, quand elle est comparée à la séquence de la SEQ ID n° : 1.

6. MVA recombinant selon l'une quelconque des revendications 1 à 5, dans lequel la séquence des paragraphes (i), (ii) et/ou la sous-séquence du paragraphe (iii) comprennent les nucléotides 25 à 29 et/ou 22 à 29 de la SEQ ID n° : 1.

7. MVA recombinant selon la revendication 1, dans lequel le promoteur ATI a la séquence de la SEQ ID n° : 1.

8. MVA recombinant selon l'une quelconque des revendications 1 à 7, dans lequel le MVA est choisi parmi : une souche MVA-BN, déposée auprès du "European Collection of Cell Cultures" (ECACC) sous le numéro V00083008, ou un dérivé de celle-ci ; et la souche MVA 575, déposée sous le numéro V00120707 auprès du ECACC.

9. MVA recombinant selon l'une quelconque des revendications 1 à 8, dans lequel la cassette d'expression est insérée dans un site de délétion existant naturellement du génome du MVA, en ce qui concerne le génome de la souche Copenhagen du virus de la vaccine ou une région inter-génique du génome du MVA.

10. MVA recombinant selon l'une quelconque des revendications 1 à 9, dans lequel la séquence codante code pour au moins un antigène, un épitope antigénique et/ou un composé thérapeutique.

11. MVA recombinant, selon l'une quelconque des revendications 1 à 10, comme vaccin ou médicament.

12. Vaccin ou composition pharmaceutique comprenant un MVA recombinant, selon l'une quelconque des revendications 1 à 10.

13. Utilisation du MVA recombinant, selon l'une quelconque des revendications 1 à 10, pour la préparation d'un vaccin ou d'un médicament destiné à provoquer une réponse immunologique contre l'agent à partir duquel est dérivé l'antigène/épitope codé par la séquence codante.

14. Utilisation selon la revendication 13, dans laquelle l'antigène/épitope fait partie d'un polypeptide, peptide ou protéine dérivé d'un virus, d'une bactérie ou d'un champignon.

15. Utilisation selon la revendication 14, dans laquelle le virus est choisi parmi le VIH, le VLTH, le virus Herpes, le virus de la Dengue, le virus de la polio, le virus de la rougeole, le virus des oreillons, le virus de la rubéole et les virus de l'hépatite.

16. Vaccin ou composition pharmaceutique, selon la revendication 12, ou utilisation, selon l'une quelconque des revendications 13 à 15, dans laquelle le vaccin, la composition pharmaceutique ou le médicament comprend au moins une TCID₅₀ de 10² (dose infectieuse en culture tissulaire) du virus.

17. vaccin ou composition pharmaceutique, selon l'une quelconque des revendications 12 à 16, ou utilisation, selon l'une quelconque des revendications 13 à 15, dans laquelle le vaccin, la composition pharmaceutique ou le médicament est à administrer en quantités efficaces d'un point de vue thérapeutique lors d'une première inoculation ("inoculation d'amorçage") et lors d'une deuxième inoculation ("inoculation de rappel").

18. Procédé d'introduction d'une séquence codante dans des cellules cibles, comprenant l'infection des cellules cibles par le virus, selon l'une quelconque des revendications 1 à 10, dans lequel la cellule cible n'est pas une cellule du corps humain et/ou animal.

19. Procédé de production d'un peptide, d'une protéine et/ou d'un virus comprenant :
a) l'infection d'une cellule hôte par le virus, selon l'une quelconque des revendications 1 à 10,
b) la culture de la cellule hôte infectée dans des conditions appropriées et
c) l'isolement et/ou l'enrichissement du peptide et/ou de la protéine et/ou des virus produits par ladite cellule hôte.

20. Cellule contenant le virus selon l'une quelconque des revendications 1 à 10.

21. Utilisation du promoteur ATI de la vaccine ou un dérivé de celui-ci, comme cela est défini dans l'une quelconque des revendications 1 à 7, pour l'expression de séquences codantes dans un MVA.

22. Procédé de production d'un MVA recombinant, selon l'une quelconque des revendications 1 à 10, comprenant l'étape consistant à insérer une cassette d'expression dans le génome d'un MVA.


### SEQUENCE LISTING

<110> Bavarian Nordic A/S
<120> Expression of genes in Modified Vaccinia virus Ankara by using the Cowpox ATI promoter
<130> BN51PCT
<150> DK PA 2002 01813
   <151> 2002-11-25
<160> 1
<170> PatentIn version 3.1
<210> 1
   <211> 29
   <212> DNA
   <213> Cowpox virus
<220>
   <221> promoter
   <222> (1)..(29)
   <223>
<400> 1
   gttttgaata aaattttttt ataataaat 29